# EUROPEAN PATENT APPLICATION

(11) **EP 4 275 713 A1**
(43) Date of publication of application: **15.11.2023**
(21) Application number: 22173009.6
(22) Date of filing: 12.05.2022
(51) Int. Cl.: A61M 5/142, A61M 5/20

(54) **MONITORING OF A DRUG DELIVERY DEVICE**

(71) Applicant: Ypsomed AG, 3401 Burgdorf (CH)
(72) Inventor: SCHÜPBACH, Simon, 3400 Burgdorf (CH); BRÜGGER, Martin, 3065 Bolligen (CH)
(74) Representative: Kleiner, Stefan

(57) **Abstract**

The invention relates to a drug delivery device (1) for dispensing a drug from a reservoir. The delivery device (1) comprising a monitoring unit (4) adapted to monitor a device parameter of the delivery device (1), wherein the delivery device (1) can be in a non-activated state and wherein the delivery device (1) can be in an activated state. The delivery device (1) further includes a trigger means connected to the monitoring unit and adapted to initiate a trigger signal based on a detected electromagnetic field or environmental parameter, or based on an elapsed time duration of a timer, wherein the monitoring unit (4) is configured to automatically switch, based on the trigger signal, the delivery device (1) from the non-activated state to the activated state and to determine subsequently a device parameter value and an instantaneous delivery device condition based on the determined device parameter value

## Description

### FIELD OF THE INVENTION

The present invention relates to medicament delivery devices for injecting, delivering, administering, infusing or dispensing substances and/or liquids such as insulin, hormone preparations or vaccines. It departs from a drug delivery device with a monitoring unit configured to monitor a device parameter of the drug delivery device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. Alternatively, in a predefined fix dose may be set and the user can administer the fix dose. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from a monitoring module or electronic unit embedded or integrated in the delivery device, or being part of an auxiliary or supplemental electronic module or add-on device releasably attachable to the delivery device. The monitoring module monitors a drug delivery process, in order to proactively prevent false handling of the device and/or to keep track of the doses already applied, and generates data related to an instantaneous condition and/or use of the delivery device. In addition, monitoring modules known in the art may monitor a parameter in the dispensing mechanism or in an electronic circuit of the drug delivery device.

WO08067314 A2 discloses an infusion pump adapted to transfer information to and from the infusion pump through an interface to a computer, or alternatively, over the Internet to a remote server, for storage. An acceleration sensor is included within the pump and is used as an indicator of potential damage to the pump due to an impact. Varying acceleration threshold levels can be programmed into the pump for each of the axes or combination of axes to produce the appropriate alarm or warning messages. The pump can alarm and instruct the user to investigate damage, such as a leaking infusion set or broken reservoir, a cracked pump housing or damage to the power supply or other electronic components. The pump can also automatically run a self-check to identify damage that may not be visible to the user.

US8585647 B2 discloses an infusion device in form of a drug pump used for a diabetes mellitus therapy. The pump is adapted to carry out a battery test during regular operation of the pump determining, based on an off-circuit voltage and the internal resistance as determined in a carried-out battery test, a capability of the battery for further powering the pump, and providing an alert to the device user in case of a lack of capability of the battery for further powering the device. During use a testing unit carries out tests in a fixed testing interval of some minutes. Therefore, the testing unit may comprise a dedicated timer. The tests may be triggered via timers and/or clock circuitry of the general circuitry.

These prior art approaches focus on tests during operation. The start is triggered by a user action, or by a predefined test interval during use of the device. Accordingly, defects or malfunctions already present before use are not detected before the patient intends to put into operation the device.

### DESCRIPTION OF THE INVENTION

It is an objective of the invention to improve an immediate operational readiness of a drug delivery device.

This objective is achieved by a drug delivery guidance system, a computer program product or a method according to the independent claims. Preferred embodiments are evident from the dependent claims.

The invention relates to a drug delivery device for dispensing a liquid drug from a reservoir in the delivery device. The drug delivery device comprises a dispensing mechanism and an electronic controller to control or to drive an actuator of the dispensing mechanism for dispensing the drug. The delivery device further includes a monitoring unit adapted to monitor, sense or detect a device parameter of the delivery device, preferably of the dispensing mechanism or of the electronic controller.

The delivery device has, and can be in, a non-activated state during a shipping, shelf, storage or non-use period in which state the electronic controller is not enabled to control or drive the actuator, and preferably is in a low power state. The delivery device has, and can be in an activated state in which the electronic controller is enabled to control or drive the actuator of the dispensing mechanism.

The delivery device includes further a trigger means (e.g. a detector or sensor) connected to the monitoring unit and adapted to initiate a trigger signal based on a detected electromagnetic field or a change of an electromagnetic field, on a detected environmental parameter or a change thereof, or an elapsed time duration of a timer. The monitoring unit is configured to automatically switch, based on the trigger signal, the delivery device from the non-activated state to the activated state and to determine subsequently a device parameter value and an instantaneous delivery device condition based on the determined device parameter value.

The drug delivery device according to the invention allows to conduct a self-check, self-test or a verification of a determined device parameter absent any user interaction. Hence, contrary to prior art approaches the test or verification of a device parameter is not triggered by a user action (switching on the device, starting an administration) nor is there a need to keep the device in an activated state or an active operation mode.

According to the invention the determination of a condition of the drug delivery device, e.g. if all function of the delivery device are available (for example a dispensing movement, power level, updated software) is tested, checked or verified well before an administration event with the drug delivery device. Namely, the self-test or verification is carried out if, for example, an environmental parameter changes or is out of a predefined range (for example humidity, temperature, air pressure) or if an (automated) self-test command is sent via electromagnetic field (for example via NFC) or if a predefined time duration is elapsed.

Therefore, in case an error, malfunction or an elapsed expiry date of a drug or device component is detected during the self-test a notification can be displayed on the device or the notification can be transmitted to a user of the device or to any external receiver well before an upcoming delivery event. Thus, the device can be replaced or a test routine can be conducted in due time. As an example, if the humidity in a storage room exceeds a threshold the monitoring unit automatically starts a dispensing motor test to verify whether or not the delivery device can still be used.

Hence, it is ensured that the drug delivery device is fully operational when the device is needed for a drug delivery, e. g. when the user takes the device out of a box, refrigerator or a storage place after a prolonged time of non-use. This is an important advantage over known approaches providing selftests only just before or during the use of the delivery device.

The delivery device may be an injection device or an infusion device. In both cases, the delivery device includes a dispensing mechanism, preferably an automatic dispensing mechanism driven by an actuator such as, for example, an electric motor, a spring or an electromagnetic drive. The electronic controller may release or drive and/or control the actuator of the dispensing mechanism.

The electronic controller is part of an electronic circuit inside the delivery device. The electronic controller may be implemented, for example in form of a microprocessor or FPGA. The controller is configured to control and drive an actuator of the dispensing mechanism and preferably configured to control or drive other electronic features of the delivery device such as power management, system update, display means, data processing and if present a communication unit.

Furthermore, the drug delivery device includes a monitoring unit configured to monitor, determine or measure a device parameter of the delivery device. Thus, the monitoring unit may include sensors to determine the device parameter value. Non limiting examples of device parameters are a power consumption of an electric actuator of the dispensing mechanism, a power level of a power source in the delivery device, a current, voltage or resistance in the electronic circuit or a software version of the electronic circuit or of a computing device in the drug delivery device.

In the present description the term "automatically" means the monitoring unit can initiate an action with the drug delivery device on its own motion without any human input. That means for determining the delivery device parameter value there is no need to switch on the device, press a button or activate any component or mechanism in the delivery device by a user.

The monitoring unit is configured to automatically determine an instantaneous (current) delivery device condition based on the determined or measured device parameter value. Preferably, the device condition is determined based on a comparison between the determined device parameter value and a reference value or a reference value range. The device condition may be indicative, for example, whether the device is fully, partly or not operational or if one or more function of the device are enabled or disabled or the condition may represent a status of the delivery device, for example, a current version of the electronic circuit or of a software of the delivery device.

In the present description a singular form is used for the device parameter value. However, it is noted that the monitoring unit can automatically determine, upon receipt of the trigger signal, more than one device parameter, preferably two or more device parameter values can be determined sequentially during a check procedure started after the monitoring unit has switched the delivery device to the activated state.

The delivery device can be in a non-activated in which the device is not ready to use and is stored, preferably for a prolonged time such as a few days, weeks or even years. In the non-activated state the electronic controller (and preferably the whole electronic circuit) is disabled, shut down or deactivated and thus does consume only very little electric energy. Hence, in the non-activated state the electronic controller is not enabled to control a dispensing or/and drive of the actuator of the dispensing mechanism. However, it is to be understood that in the non-activated state the trigger means (environmental sensor, electromagnetic circuit or timer) and its software and/or electronics are adapted to run and thus are operational even in the non-activated state.

In the activated state the delivery device is ready to be used or is in use. That means in the activated state the electronic controller and the electronic circuit are enabled to control a dispensing and to drive the actuator of the dispensing mechanism. The electronic circuit consumes more electric energy compared to the non-activated state.

The drug delivery device comprises at least one trigger means (or detector) in form of an environmental sensor, an electromagnetic field detection circuit (e. g. a communication circuit) or a timer adapted to run a predefined time duration. The permanently active trigger means initiates or generates a trigger signal or input for the monitoring unit based on a detected electromagnetic field (or change thereof), a detected environmental parameter (or change thereof) or an elapsed time duration of the timer.

The environmental sensor is a sensor adapted to sense an environmental parameter such as, for example, humidity, temperature, air pressure, particles, odors or smoke.

If the device includes a timer the timer runs after a start a predefined time duration. The duration lasts preferably at least one day but may last, for example, several days, weeks, months or years. The timer may be started initially after manufacturing of the delivery device. Alternatively, the timer may be started or restarted automatically by the electronic controller after a predefined event, for example, after a first time use of the delivery device or after a previous time duration of the timer. Furthermore, the controller may revert to a calendar and generate the trigger at regular intervals.

The timer may be implemented as a separate hardware or it may be implemented as a software feature providing a timer function. An internal clock such as a real time clock (RTC) may provide a trigger for the timer or may provide the timer function.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the monitoring unit is configured to automatically switch, after determining the device condition, the delivery device from the activated state in the non-activated state or in a sleep mode or power-save mode. Hence, the device is only in the activated state for determining the device parameter value and the device condition and potentially for providing a user notification. The delivery device is subsequently switched to a low power mode to the non-activated state. This provides an efficient power management of the drug delivery device and ensures that the device does not consume power during storage or during a non-used period.

The switch back to the non-activated state may depend on the determined delivery device condition. That means, for example, if an error or malfunction is detected the monitoring unit may keep the device in the activated state (for example for sending periodically notifications) until the user confirms the notification, switches the device off manually or resolves the detected error.

The trigger means (detector) of the drug delivery device is preferably one of a temperature sensor, a humidity sensor, a pressure sensor, a particle sensor or an odor or smoke sensor. Such sensors can provide a sensor signal which can be used to initiate or generate the trigger signal for the monitoring unit. By way of example, if a sensor signal exceeds a predefined threshold the trigger signal may be initiated or generated. For example, the trigger signal is generated if a temperature level is above or below a temperature reference value or a humidity level exceeds a reference level.

The drug delivery device preferably includes a communication unit adapted to establish a wireless data communication from the drug delivery device to an external device. The latter may be, for example, a cloud server, a user mobile device or a computing device of a health care practitioner (HCP), a healthcare institution, a drug manufacturer or a delivery device manufacturer.

The communication unit can send the device parameter value or information relating to the determined device condition to the external device. The communication may be performed, for example, by a mobile device communication standard such as LTE-M, a wireless wide area network (WAN) such as NB-IoT or a local area network (WLAN) or by another short or near range wireless communication technology such as Bluetooth, ZigBee, WiMAX or NFC.

The data communication is advantageous to remotely inform a user, HCP or manufacturer of a detected error condition of the delivery device or to confirm that the device is operational and thus is ready for an upcoming drug delivery event.

Preferably, the monitoring unit is configured to compare the determined device parameter value with a reference value or reference range and to automatically send a notification to the external device based on a result of the comparison. Thus, the user or any external receiver may be informed if the determined device parameter value exceeds a predefined threshold, e. g. the device does not work according to a defined scheme or does not any more fulfil all requirements correctly.

In a preferred embodiment the monitoring unit exclusively sends the notification if the determined parameter value is not in accordance with the reference value (e.g. exceeds a predefined threshold) or if the determined parameter value lies outside a predefined reference value range. Hence, the user or any external receiver is only informed when a device parameter value is above, below a threshold or lies outside an acceptable predefined range.

This allows to monitor a device condition remotely and thus even if the device is stored in a storage facility a long distance away from the user, HCP, distributor or manufacturer these receivers are informed and up-to-date about a current condition of the delivery device

Furthermore, the drug delivery device preferably includes a positioning module and the monitoring unit is configured to send positional data from the positioning module to the external receiver. Examples of a positioning modules are a global navigation satellite system (GNSS) module such as a GPS module or an indoor positioning system (IPS) module.

An error report or a positive result of a function test may be send along with position data to the external receiver. This may help the external receiver to identify the drug delivery device and may facilitate a potential error handling. That is, a user, HCP or manufacturer is able to locate the device and if necessary can easily find the device (for example within several storage places) to replace the delivery device or to repair it.

The dispensing mechanism of the drug delivery device includes an actuator and the monitoring unit is preferably configured to drive the actuator of the delivery device for an operational self-test, upon receipt of the trigger signal, and wherein the device parameter value is an actuator parameter value measured during driving the actuator for the self-test.

The actuator is driven to determine the condition and function of the actuator and/or the whole dispensing mechanism, preferably no drug is dispensed from the reservoir during this test.

Examples of an actuator are an electric motor, a gas actuator, an electromagnetic coil actuator or a pretensioned resilient element (spring, elastomer) which can be automatically released by the monitoring unit, for example, by means of an electromagnet. The actuator parameter determined or measured during driving the actuator is indicative of an operational function of the actuator or the dispensing mechanism.

The actuator parameter value may be at least of one of an electric power consumption or voltage level, a resistance value, an actuator power value, a back-emf value or an actuator encoder value.

Alternatively or in addition to the above described test, the monitoring unit may be configured to check a current software version. In this case the device parameter value or one of a plurality of determined device parameter values is a software version value of the electronic circuit or a computing device in the delivery device. The monitoring unit is configured to automatically request a software update depending on a result of a comparison of the determined software version value with a predefined reference version value. The reference value may be stored in a memory in the monitoring unit or it may be previously transmitted from an external device to the monitoring unit.

A potential software download form an external computing device such as a cloud server may be initiated by the monitoring module automatically. The software version check ensures that the device is equipped with the latest software and is ready to use for an upcoming delivery event.

Furthermore, the monitoring unit may determine the following delivery device parameter values instead of or in addition to the above descripted actuator parameter and software version. Such further device parameter values may be one of a power level of a power source in the delivery device, an electronic parameter of the electronic circuit (current, voltage, resistance) or an expiry date of the drug inside the drug delivery device.

If the delivery device includes a timer the lattre is preferably configured to run for a first time duration after a manufacturing of the drug delivery device. That means the timer is started after a final assembly of the drug delivery device, preferably at the manufacturer's site. Thus, the delivery device is tested after a predefined time duration after manufactured, for example, before delivered to the end user or distributor and/or after an initial shelf time. Thus, manufacturing defects can be reliably detected.

In a preferred embodiment the monitoring unit is configured to automatically restart the timer to run for a second time duration after elapse of the first time duration without any manual user interaction. That means after determining the device condition the timer is started again. Hence, a periodic determination of the delivery device condition is provided. This may be in particular advantageous for quickly changing device parameters or parameters that indicate an expiry date, such as a power level or the validity of a drug. A corresponding notification can be provided indicating, for example, that a power level is going to be soon below a critical threshold.

The monitoring unit may restart the timer more than once and may thus provide a periodic monitoring of the delivery device condition. By way of example, the device condition based on the measured device parameter may be determined every day, every month or only a few times a year.

The first, second or any further time duration preferably depends on a determined delivery device condition after an initial test or after the first or a previous time duration. The second time duration can thus be chosen to be longer or shorter than the first or a previous time duration depending on an instantaneous delivery device condition. By way of example, the second time duration may be shorter if a low battery level is detected or if the drug expires within the next few days.

The variable time duration thus allows to inform a user in due time of a changing device condition and thus the user can take necessary steps such as a replacement, a repair or adjustment of the device. On the other hand the delivery device can be kept in the shelf as long as it is fully operational or/and is not yet out-of-date.

The invention further relates to a method for automatically determining a drug delivery device condition, the method including the steps of
a. Detecting at least one of an electromagnetic field or a change of the filed, an environmental parameter or a change of the parameter or an elapsed time duration of a timer;
b. Initating, based on the detected electromagnetic field, the environmental parameter or the elapsed time, a trigger signal;
c. Automatically switching, upon receipt of the trigger signal, the delivery device from a non-activated state in which a delivery device electronic controller is not enabled to control an actuator of the delivery device in an activated state in which the electronic controller is enabled to control the actuator;
d. Determining a device parameter value of the drug delivery device (1);
e. Deriving an instantaneous delivery device condition based on the determined device parameter value.

Furthermore, the invention relates to a computer program comprising instructions which, when executed by a computing device of the drug delivery device, cause the computing device to carry out the method as described above.

The computing device in the drug delivery device can be a microcontroller, FPGA, DSP or the like. The computing device is connected to sensing means of the monitoring unit which are adapted to determine the device parameter value.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: schematically depicts a monitoring arrangement for a drug delivery device according to the invention;
- Fig. 2: depicts a drug delivery device in form of an automatic autoinjector with a motor;
- Fig. 3: depicts a flow diagram of a motor self-test and
- Fig. 4: depicts a flow diagram of a software update self-check.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

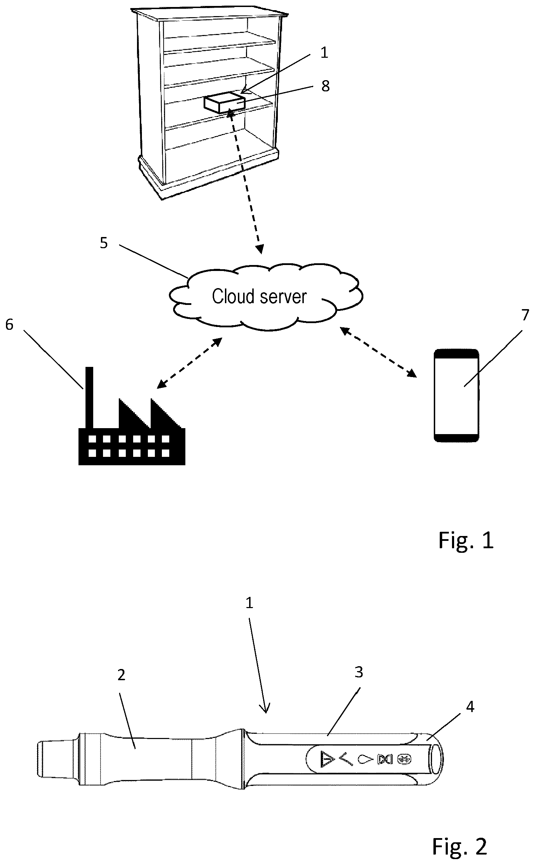

Figure 1 schematically depicts a monitoring arrangement with a drug delivery device according to the invention. The delivery device in form of a semi-reusable autoinjector 1 is wirelessly connected to a cloud server 5. The autoinjector 1 is not visible in figure 1 as it is in a storage box 8. A manufacturer or supplier 6 of the autoinjector 1 has access to cloud data provided by the cloud server 5. Furthermore, a user has access to cloud data via user mobile device in form of a mobile phone 7.

Although not shown a health care practitioner, a healthcare insurance and/or a drug manufacturer can be connected to the cloud server 5 and may have access to the drug delivery data.

The monitoring arrangement according to the invention is descripted herein with the semi-reusable autoinjector 1 shown in figure 2 and including an electric motor for automatically dispensing a liquid drug from a cartridge. However, the monitoring can be applied to other delivery devices which include the descripted monitoring unit as will be described below. In other embodiments the delivery device may be an infusion drug pump, a patch injector or a fully reusable electronic injection pen with an electric actuator.

The semi-reusable autoinjector 1 as shown in figure 2 comprises a cartridge or syringe unit 2 as disposable part and a drive unit 3 as reusable part. The latter can be reused for several injections. The syringe unit 2 comprises a reservoir in form of the syringe with the liquid drug, and a syringe holder. The drive unit 3 comprises a dispensing mechanism with a plunger rod (not shown) adapted to move a piston inside the syringe in a dispensing direction to dispense the liquid drug from the syringe. The drive unit 3 further includes a brushed DC motor for moving the plunger rod and electronics including a controller and a monitoring unit 4 for sensing parameters of the dispensing mechanism or of the electronics in the drive unit 3. The injector further includes a humidity sensor, a communication module for establishing a data connection to the external cloud server 5, a GPS module and a power source in form of a battery (all not shown) for supplying electrical energy to the electrical consumers inside the autoinjector 1.

In the following the function of the monitoring is descripted in detail.

The autoinjector 1 includes an electronic circuit with a real time clock (RTC) including a timer function. The timer runs a predefined time duration, for example 30 days. After assembly of the autoinjector the timer is started for a first time at the manufacturer's site.

The autoinjector 1 can be in two states, in a power save state or power save mode and in an activated state. In the power save state the activities of the electronic circuit are reduced to a minimum allowing only the running of the timer (RTC) and the operating of the humidity sensor.

All other functions and activities of the electronics and of the controller are disabled in the power save state. In particular, a driving of the motor and thus a dispensing is not enabled in the power save state. In the activated state the autoinjector 1 is ready to use. That means the electronic circuit and the controller are enabled to control a movement the motor. The timer and the humidity sensor are operational independent of the injector's state and without any user input.

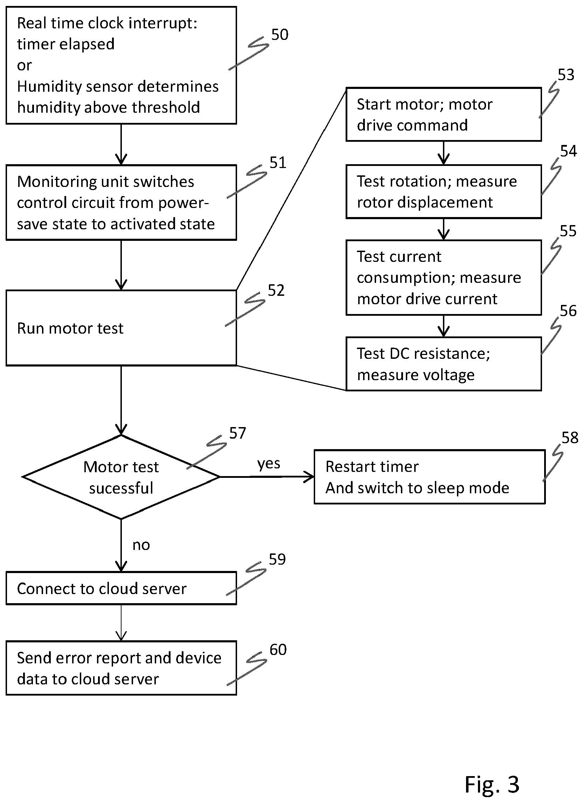

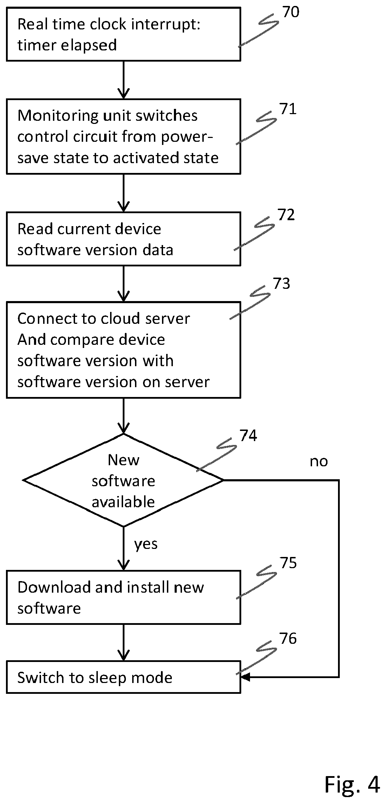

After the predefined time duration of the timer is elapsed a motor self-test and a software update check are automatically started by the monitoring unit or more precisely by a computing device in form of a microprocessor of the monitoring unit as described below with respect to figures 3 and 4.

Independent of the timer the humidity sensor senses a current atmospheric humidity. If the determined humidity exceeds a predefined threshold, for example if the humidity is above 80% a trigger is generated for the monitoring unit to start automatically the motor self-test independent of the timer.

As shown in the flow diagram of figure 3 the motor self-test procedure starts with a trigger initiated either by the timer or the humidity sensor. After elapse of the time duration or if the humidity sensor has detected a humidity above the threshold at step 50 the electronics of the injector are activated to switch from the power save state to the activated state (step 51). Subsequently, the monitoring unit runs the motor test (step 52) to verify whether the electric motor inside the injector is fully operational.

The motor self-test starts with a motor drive command at step 53 moving a rotor of the motor at least one full rotation. The monitoring unit captures the signal of a motor encoder to verify if the rotor follows the drive command at step 54. Additionally, during rotation of the rotor the consumed motor drive current is sensed by the monitoring unit at step 55. The motor test ends with a DC resistance test by sensing a DC voltage in the stator. Subsequently, the monitoring unit evaluates at step 57 all measured injector parameter values (encoder value, drive current value, voltage value) and determines whether or not the stepper motor is fully functioning.

In case the motor is fully operational and thus the motor test was successful the timer is restarted for another time duration and the electronics are switches from the activated state in the power save state (step 58). If the injector is not used by the user, for example, if the injector is in a storage place the electronics are kept in the power save mode upon elapse of the time duration of the timer or upon the humidity sensor detects a humidity above the threshold.

In case the monitoring module has determined that the motor does not work correctly a wireless data connection to the external cloud server 5 is established by the communication unit at step 59. Subsequently, the monitoring unit sends an error report with information about the detected failure in the motor along with injector device specific data (serial number, device type, date) and GPS data to the cloud server (step 60).

If the injector 1 is in a storage place of the user the cloud server 5 sends a notification to the user mobile device 7 to inform the user about the detected error. The user can thus order a new injector in due time before a planned administration. Additionally, a notification can be sent from the cloud server 5 to the manufacturer or supplier 6 of the injector to keep record of failures of the injector. Based on the injector specific data and GPS data from the GPS module the manufacturer can locate the injector and determine the production batch. Suitable steps such as a replacement or callback can be initiated.

Referring to figure 4 a software update check automatically started by the monitoring unit will be described in detail. The software check can be carried out instead of the above described motor self-test. In a preferred embodiment the software test is conducted in addition to the motor self-test.

The procedure is initiated by a timer after elapse of a time duration (step 70) as described above. The injector is switched from the power save state in the activated state at step 71 and the monitoring unit determines the current software version or reads the current software version from a memory in the injector at step 72. At step 73 the monitoring unit establishes via communication unit a data connection to the cloud server and verifies whether the latest available software version is installed in the injector by comparing the injector software version with the software version on the cloud server (step 74).

If the latest version is installed in the injector the timer is restarted for a further time duration and the electronics are switched to the power save state.

If the verification reveals that the injector software version is not the latest version the monitoring unit automatically starts a download of the latest version and installs the new software in the injector (step 75). Subsequently, the injector is switch to the power save state at step 76.

In another embodiment the communication module of the injector includes an active RFID transponder adapted to receive a command from an external computer via NFC communication or via any radio frequency such as UHF and is adapted to trigger the monitoring unit. Upon receipt of the command the monitoring unit automatically switches the injector from the power save state to activated state and starts a function test with injector, for example, including the above described motor self-test. After the self-test the injector is switched to the power save state.

That means in a storage room or in a distribution centre where a plurality of injectors are stored a remote central computer can wirelessly send self-test commands to all injectors. These commands can be sent automatically by the central computer on a regular basis and thus allowing to perform periodically a test procedure with all stored injectors.

In a further embodiment, the monitoring unit additionally checks the power level of the battery in the injector upon elapse of the time duration. If it is determined that the battery level is below a predefined minimum, for example, less than 15% of the total battery capacity the monitoring unit connects to the cloud server and a notification is sent to the user to allow a replacement of the battery in due time.

If the battery level is below 25% of the total capacity a notification is not issued but the next time duration for the timer is shortened, for example, from 30 days down to 10 days. That means the interval for the self-test is shorter. Alternatively, the time duration can be extended in order to save electrical energy after a low battery level has been determined.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

## Claims

1. A portable or wearable drug delivery device (1) for dispensing a drug from a reservoir, the delivery device (1) comprising
a dispensing mechanism and an electronic controller to control an actuator of the dispensing mechanism for dispensing the drug;
a monitoring unit (4) adapted to monitor a device parameter of the delivery device (1), wherein the delivery device (1) can be in a non-activated state during a non-use period in which state the electronic controller is not enabled to control the actuator and
wherein the delivery device (1) can be in an activated state in which state the electronic controller is enabled to control the actuator,
**characterized in that** the delivery device (1) further includes
a trigger means connected to the monitoring unit and adapted to initiate a trigger signal based on a detected electromagnetic field or environmental parameter, or based on an elapsed time duration of a timer,
wherein the monitoring unit (4) is configured to automatically switch, based on the trigger signal, the delivery device (1) from the non-activated state to the activated state and to determine subsequently a device parameter value and an instantaneous delivery device condition based on the determined device parameter value.

2. Drug delivery device (1) according to claim 1, wherein the monitoring unit (4) is configured to automatically switch, after determining the device condition, the delivery device (1) from the activated state to the non-activated state.

3. Drug delivery device (1) according to claim 1 or 2, wherein the trigger means is one of a temperature sensor, a humidity sensor, a pressure sensor, a particle sensor or an odor or smoke sensor.

4. Drug delivery device (1) according to any of claims 1 to 3, wherein the delivery device (1) comprises a communication unit adapted to establish a wireless data communication from the delivery device (1) to an external device (5).

5. Drug delivery device (1) according to claim 4, wherein the monitoring unit (4) is configured to compare the device parameter value with a reference value or reference range and to automatically send a notification to the external device based on a result of the comparison.

6. Drug delivery device (1) according to claim 4 or 5, further comprising a positioning module and wherein the monitoring unit (4) is configured to send positional data to the external device (5).

7. Drug delivery device (1) according to any of claims 1 to 6, wherein the monitoring unit (4) is configured to drive the actuator of the delivery device, based on the trigger signal, and wherein the device parameter value is an actuator parameter value measured during driving the actuator.

8. Drug delivery device (1) according to claim 7 wherein the actuator parameter value is at least indicative of one of a power consumption or voltage change, an actuator power value, a back-emf value or an actuator encoder value.

9. Drug delivery device (1) according to any of claims 1 to 8, wherein the device parameter value is a software version value of the electronic controller or a computing device in the delivery device and wherein the monitoring unit is configured to request a software update depending on a result of a comparison of the software version value with a predefined reference value.

10. Drug delivery device (1) according to any of claim 1 to 8, wherein the device parameter value is one of a power level of a power source in the delivery device, an electronic parameter or an expiry date of the drug inside the drug delivery device.

11. Drug delivery device (1) according to any of claims 1 to 10, wherein the timer is configured to run for a first time duration after a manufacturing of the drug delivery device

12. Drug delivery device (1) according to claim 11, wherein the monitoring unit (4) is configured to restart the timer to run for a second time duration after elapse of the first time duration.

13. Drug delivery device (1) according to claim 12, wherein the second time duration depends on the determined device condition after the first time duration.

14. Method for automatically determining a drug delivery device (1) condition, the method including the steps of
a. Detecting at least one of an electromagnetic field, an environmental parameter or an elapsed time duration of a timer;
b. Initating, based on the detected electromagnetic field, the environmental parameter or the elapsed time, a trigger signal;
c. Automatically switching, upon receipt of the trigger signal, the delivery device (1) from a non-activated state in which a delivery device electronic controller is not enabled to control an actuator of the delivery device in an activated state in which the electronic controller is enabled to control the actuator;
d. Determining a device parameter value of the drug delivery device (1);
e. Deriving an instantaneous delivery device condition based on the determined device parameter value.

15. Computer program comprising instructions which, when executed by a computing device of the drug delivery device (1), cause the computing device to carry out the method according to claim 14.
